(19) Europäisches Patentamt
European Patent Office
Office européen des brevets

(11) Veröffentlichungsnummer: **0 444 292 A2**

(12) # EUROPÄISCHE PATENTANMELDUNG

(21) Anmeldenummer: **90124851.8**

(22) Anmeldetag: **20.12.90**

(51) Int. Cl.5: **C08L 77/00**, C07C 69/88, C07C 69/90, C09K 19/20, C08K 5/10

(30) Priorität: **01.03.90 DE 4006404**

(43) Veröffentlichungstag der Anmeldung: **04.09.91 Patentblatt 91/36**

(84) Benannte Vertragsstaaten: **DE FR GB IT**

(71) Anmelder: **BAYER AG**
**W-5090 Leverkusen 1 Bayerwerk(DE)**

(72) Erfinder: **Pielartzik, Harald, Dr.**
**Doerperhofstrasse 39**
**W-4150 Krefeld(DE)**
Erfinder: **Heinz, Hans-Detlef, Dr.**
**Breslauerstrasse 31**
**W-4150 Krefeld(DE)**
Erfinder: **Dhein, Rolf, Dr.**
**Deswatinesstrasse 30**
**W-4150 Krefeld(DE)**
Erfinder: **El Sayed, Aziz, Dr.**
**Saarlauterner Strasse 39**
**W-5090 Leverkusen 1(DE)**

(54) **Leichtfliessende Polyamid-Formmassen.**

(57) Die Erfindung betrifft leichtließende Polyamid-Formmassen, gekennzeichnet durch einen Gehalt an neuen flüssigkristallinen Verbindungen der allgemeinen Formeln (I) und/oder (II) sowie die neuen flüssigkristallinen Verbindungen der Formeln (I) und (II).

EP 0 444 292 A2

Die Erfindung betrifft leichtließende Polyamid-Formmassen, gekennzeichnet durch einen Gehalt an neuen flüssigkristallinen Verbindungen der allgemeinen Formeln (I) und/oder (II) sowie die neuen flüssigkristallinen Verbindungen der Formeln (I) und (II).

Polyamide sind eine seit vielen Jahren für eine Vielzahl praktischer Anwendungen bewährte Klasse von Polymeren, die nach verschiedenen Verfahren hergestellt werden können, die aus sehr unterschiedlichen Polyamid-bildenden Bausteinen synthetisiert werden können und die im speziellen Anwendungsfall, allein oder auch in Kombination mit Verarbeitungshilfsmitteln, polymeren Legierungspartnern oder auch mineralischen Verstärkungsmaterialien (wie z.B. Füllstoffe oder Glasfasern), zu Werkstoffen mit speziell eingestellten Eigenschaftskombinationen ausgerüstet werden können. So werden Polyamide in großen Mengen zur Herstellung von Fasern, Kunststoff-Formteilen und Folien, aber auch z.B. als Schmelzkleber und Hilfsmittel in einer Vielzahl von Anwendungen technisch eingesetzt.

Ein sehr großer Teil der verschiedenen Polyamid-Formmassen wird im Spritzguß verarbeitet, d.h. das Polyamid wird aufgeschmolzen und unter Druck in eine Form gepreßt, in der es beim Abkühlen erstarrt. Hier spielt die Fließfähigkeit eine entscheidende Rolle; je leichter eine Polyamid-Masse fließt, desto besser kann die Form gefüllt werden. Dies ist insbesondere bei sehr dünnwandigen Teilen von größter Bedeutung.

Während die teilkristallinen Polyamide mittleren Molekulargewichts eine gute Fließfähigkeit besitzen, sinkt diese infolge der mit steigendem Molekulargewicht stark wachsenden Schmelzviskosität beim Übergang zu höhermolekularen Polyamiden schnell ab. In Anbetracht der Tatsache, daß die mechanischen Eigenschaften von Polyamiden mit steigendem Molekulargewicht noch besser werden, insbesondere die Schlagzähigkeit, stellt dies eine drastische Beschränkung ihres Einsatzbereiches dar.

Im Gegensatz zu den teilkristallinen, aliphatischen Polyamiden weisen amorphe Polyamide, insbesondere dann, wenn ihre Glastemperaturen oberhalb von ca. 150° C liegen, schon bei relativ niedrigen Molekulargewichten sehr hohe Schmelzviskositäten und entsprechend geringe Fließfähigkeit auf. Auch hier ist die Verarbeitbarkeit also erschwert.

Bekanntlicherweise nehmen die teilkristallinen Polyamide, je nach Struktur, unterschiedliche Mengen Wasser auf. Dies führt zu einer Erhöhung der Zähigkeit, jedoch zu einer Verringerung der Steifigkeit, da das Wasser als Weichmacher wirkt.

Zur Erhöhung der Steifigkeit werden Polyamide daher seit langem mit anorganischen Verstärkungsstoffen, z.B. Glasfasern oder mineralischen Füllstoffen, ausgerüstet.

Die durch die anorganischen Verstärkungsstoffe erzielte Erhöhung von Steifigkeit, Härte, Wärmeformbeständigkeit und Dimensionsstabilität wird jedoch in aller Regel durch Nachteile erkauft, insbesondere durch eine verminderte Fließfähigkeit - besonders bei faserförmigen Verstärkungsstoffen - wodurch die Verarbeitbarkeit erschwert wird.

Die verringerte Fließfähigkeit hat zur Folge, daß, insbesondere für großflächige Formteile, zum Füllen der Form immer mehr Angußstellen benötigt werden, d.h. die Werkzeuge werden immer aufwendiger. Dadurch bedingt tritt auch eine größere Zahl unerwünschter Fließnähte auf, die die optischen und mechanischen Gebrauchseigenschaften der Formteile beeinträchtigen können. Weiterhin wird es sehr schwierig, einwandfreie Oberflächenqualitäten und dünnwandige Formteile herzustellen.

Es wäre daher von großer technologischer Bedeutung, wenn die Fließfähigkeit o.g. Polyamidtypen drastisch verbessert werden könnte.

Polyamid-Legierungen weisen je nach Art des polymeren Legierungspartners unterschiedliche Eigenschaften im Vergleich zu reinen PA-Komponenten auf und sind somit besonders wertvolle Werkstoffe.

Wohlbekannte Legierungen stellen z.B. die schlagzäh modifizierten Polyamide dar. Hier handelt es sich um zweiphasige Polymerlegierungen, welche zur Erhöhung der Schlag- bzw. Kerbschlagzähigkeit von Polyamiden im spritzfrischen Zustand oder auch bei tiefen Temperaturen spezielle elastomere Partner enthalten. Beispiele für solche Legierungspartner sind z.B. Dien- und Acrylatkautschuke, EPDM, Ethylen-Acrylsäure-Copolymere und andere, wie sie im Stand der Technik in großer Zahl beschrieben sind.

Zusätzlich zu dem Elastomermodifikator können Verstäfkungsstoffe anwesend sein; in solchen Legierungen sind dann sowohl Zähigkeit als auch Steifigkeit gegenüber nicht modifizierten Polyamiden erhöht.

Eine weitere große Gruppe von Polyamid-Legierungen enthält z.B. amorphe Thermoplasten mit gegenüber Standardpolyamiden erhöhter Glastemperatur und Steifigkeit.

Dadurch wird die Wasseraufnahme der Polyamide verringert und die Wärmeformbeständigkeit (gemessen z.B. durch die Heat-distortion-Temperatur (HDT)) sowie die Steifigkeit erhöht. Beispiele für Legierungspartner dieser Art sind Polystyrol, ABS, Polycarbonat, aromatische Polyester(Carbonate), Polyphenylenoxide, Polymethylmethacrylat, Polyethersulfone und andere mehr. Diese Legierungen können auch zusätzlich noch Schlagzähmodifikatoren enthalten.

Auch Legierungen von Polyamiden mit teilkristallinen Thermoplasten sind bekannt.

Von Nachteil ist allerdings, daß auch diese PA-Legierungen i.a. eine gegenüber der reinen PA-Komponente stark verringerte Fließfähigkeit besitzen und ihre Verarbeitbarkeit daher erschwert ist. Dies gilt insbesondere dann, wenn die Legierungen zusätzlich noch Verstärkungsstoffe enthalten.

Daher wäre es auch für diese Werkstoffe von großer technologischer Bedeutung, die Fließfähigkeit deutlich erhöhen zu können und somit ihre Verarbeitbarkeit zu hochwertigen Formteilen zu erleichtern. Dadurch könnte auch die Verarbeitungstemperatur gesenkt werden, was eine geringere Materialbelastung bedeutet. Dies wirkt sich besonders bei Legierungen mit Kautschukmodifikatoren positiv aus.

Überraschend wurde nun gefunden, daß spezielle flüssigkristalline, niedermolekulare und/oder oligomere Additive der allgemeinen Formel (I) und/oder (II), schon in sehr geringen Mengen den Polyamiden zugesetzt, eine drastische Erhöhung der Fließfähigkeit von Polyamid-Formmassen bewirken. Darüber hinaus kann durch diese Maßnahme auch eine Reihe mechanischer Eigenschaften sowie u.U. die Wärmeformbeständigkeit verbessert werden. Weiterhin kann durch diese Maßnahme auch gegebenenfalls die Wasseraufnahme der Polyamide verringert sowie die Kristallisationsgeschwindigkeit und der Kristallisationsgrad erhöht werden, was sich durch verbesserte Dimensionsstabilität von Formteilen und längere Verarbeitbarkeit geöffneter Gebinde, sowie u.U. geringere Zykluszeiten äußern kann.

Gegenstand der Erfindung sind daher neue, leichtfließende Polyamid-Formmassen, dadurch gekennzeichnet, daß sie durch Vermischen von

1) 85-99,9 Gew.-% an sich bekannter Polyamide mit

2) 0,1 bis 15 Gew.-%, bevorzugt 0,3 bis 8 Gew.-%, besonders bevorzugt 0,5 bis 5 Gew.-% hydroxylgruppenhaltiger, niedermolekularer, flüssig kristalliner Ester und/oder Esteramide ("LC-Fließverbesserer") des allgemeinen Strukturtyps (I)

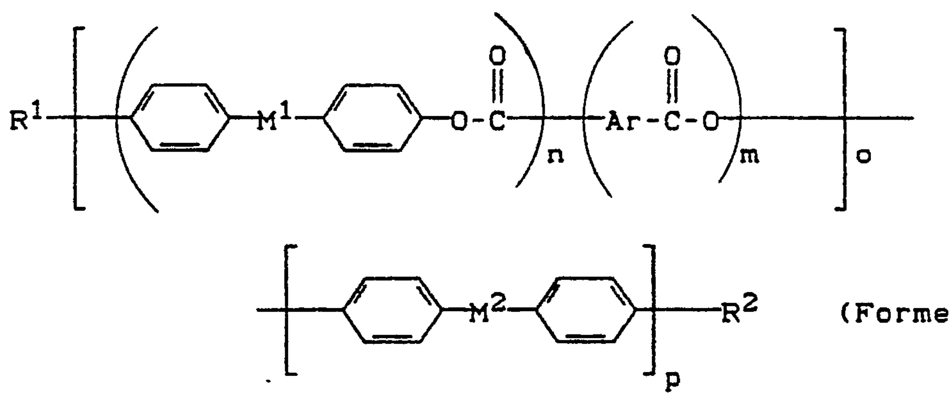

(Formel I)

worin

-Ar ein aromatischer Rest mit 6-20 C-Atomen ist, der einkernig oder mehrkernig sein kann, wobei der mehrkernige Rest über eine Bindung verknüpft oder annelliert sein kann,

worin

$R^1$ und $R^2$ gleich oder verschieden sein können und mindestens einer der Reste $R^1$ und $R^2$ für einen Rest der Formeln (III) - (XI) steht, gegebenenfalls der zweite der Reste aus $R^1$ und $R^2$ einem Hydroxygruppen-freien Rest entspricht.

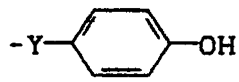
(III)

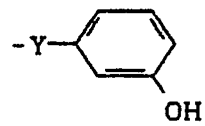
(IV)

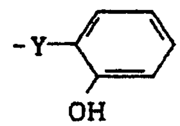
(V)

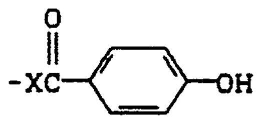
(VI)

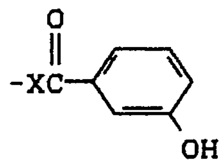
(VII)

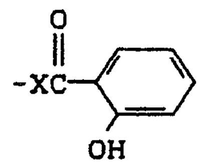
(VIII)

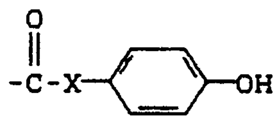
(IX)

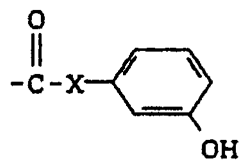
(X)

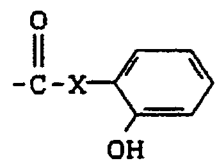
(XI)

Y eine chemische Bindung oder einen zweiwertigen Kohlenwasserstoffrest mit 1-10 C-Atomen oder auch eine $-SO_2$-Gruppe, bevorzugt eine chemische Bindung oder einen $-C(CH_3)_2$-Rest bedeutet,

X für O oder NH steht,

und wobei die aromatischen Ringe in den Formeln (III) - (XI) auch mit beispielsweise Halogenatomen oder Alkylgruppen substituiert sein können,
worin $-M^1$ und $-M^2$ gleich oder verschieden sind und zweibindige Reste der Formeln I.1) bis I.11)

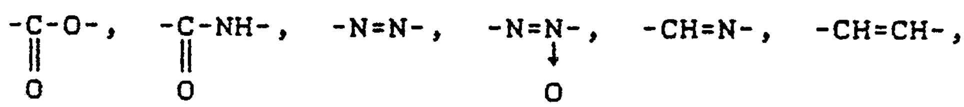

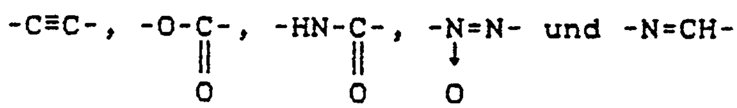
und

(Formeln (I.1 - (I.11), entsprechend ihrer Aufeinanderfolge)
sein können,
und worin

- m Null, 1 oder 2,
- n 1 oder 2,
- o 1, 2, 3 oder 4 und
- p Null oder 1 sind,

und/oder
flüssig kristalliner Ester- und/oder Esteramid-Oligomerer des Strukturtyps der Formel (II) mit statistischer Verteilung der Struktureinheiten

$$\left[R^3-\right]\left[-O-Ar^a-\overset{\overset{O}{\|}}{C}-\right]_b\left[-O-Ar^b-O-\right]_c\left[-\overset{\overset{O}{\|}}{C}-R^4-\overset{\overset{O}{\|}}{C}-\right]_d\left[-NH-R^5-Y'-\right]_e\left[-R^3\right]$$

**Formel (II)**

worin

| | |
|---|---|
| $Ar^a$ | ein bivalenter, gegebenenfalls substituierter (Substituenten wie in $R^3$), ein- oder mehrkerniger aromatischer Rest mit 6-24 C-Atomen ist, wobei der mehrkernige Rest direkt verknüpft oder auch anelliert sein kann und wobei die Carboxylgruppe auch über einen aliphatischen Rest mit dem aromatischen Ring verknüpft sein kann, |
| $Ar^b$ | einen bivalenten, gegebenenfalls substituierten, ein- oder mehrkernigen aromatischen Rest mit 6-30 C-Atomen darstellt, wobei der mehrkernige Rest unterschiedlich verknüpft sein kann, |
| $R^4$ bzw. $R^5$ | für einen Alkylrest mit 0-40 ($R^4$) bzw. 3-40 ($R^5$) C-Atomen stehen oder beide auch für einen cycloaliphatischen Rest mit 5-15 C-Atomen stehen können oder die Bedeutung eines gegebenenfalls substituierten bivalenten, ein- oder mehrkernigen aromatischen Restes mit 6-24 C-Atomen haben, wobei der mehrkernige Rest unterschiedlich über eine Bindung oder auch annelliert verknüpft sein kann, |
| Y' | für -O-, $$-\overset{\overset{O}{\|}}{C}-$$ oder -NH- steht, |
| b = | Null bis 10, vorzugsweise bis 8, insbesondere bis 4, |
| c = | 1 bis 9, vorzugsweise bis 7, insbesondere 1 bis 4, |
| d = | Null bis 9, vorzugsweise bis 7, insbesondere bis 3, |
| e = | Null bis 3, vorzugsweise bis 2, ist und |

worin

$R_3$ je nach Verknüpfung mit den anderen Bausteinen für die Reste der Formeln (XII) - (XIV),

$$-\overset{\overset{O}{\|}}{C}-Ar^a-OH \qquad (XII)$$

$-O-Ar^b-OH$ (XIII)

$-NH-R^5-OH$ (XIV)

wobei die Symbole die schon genannte Bedeutung haben, steht, und wobei das mittlere Molekulargewicht $\overline{M}n$ der Verbindungen der Formel (II) nicht größer als 4.000, bevorzugt nicht größer als 2.500 ist,

sowie gegebenenfalls

3) 0,001-150 Gew.-%, bezogen auf die Summe der Gewichte der Komponenten 1) und 2), an üblichen Zusatzstoffen, wobei die Zusatzstoffe wahlweise auch ganz oder teilweise schon in der unter 1) genannten Polyamiden enthalten sein können,

hergestellt werden.

Bevorzugt sind beide Reste $R^1$ und $R^2$ vom Hydroxylgruppen (Phenolgruppen) tragenden Typ (III) - (XI).

<u>Zu den Verbindungen der Formel (I):</u>

Besonders bevorzugte Reste -Ar- der Formel (I) und der Reste $Ar^a$ und $Ar^b$ in Formel (II) sind die Reste (Ar1) bis (Ar4)

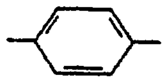 , 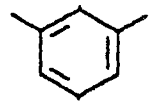 , 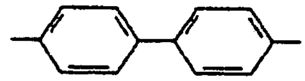 ,

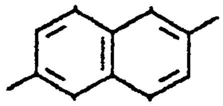

(Ar1)-(Ar4), entsprechend ihrer Reihenfolge.

Bevorzugte Reste $R^1$ und $R^2$ sind die Reste (III), (VI) und (IX).

Bevorzugte Reste -M- sind solche der Formeln (I.1), (I.2), (1.5), (I.8), (I.9) und (1.11).

Bevorzugte Indexkombinationen m, n, o, p und q sind

m = Null, n = 1, o = 1, p = Null;
m = Null, n = 1, o = 1, p = 1;
m = Null, n = 2, o = 1, p = 1;
m = n = o = p = 1;
m = 1, n = 1, o = 2, p = 1;
m = Null, n = 2, o = 1, p = Null;
m = 1, n = 2, o = 1, p = 1 und
m = 1, n = 1, o = 3, p = 1.

Die Verbindungen der Formel (I) zeichnen sich dadurch aus, daß sie beim Schmelzen eine flüssigkristalline Phase durchlaufen (bezüglich flüssigkristalliner Verbindungen bzw. Phasen siehe beispielweise: D. Demus, L. Richter, Textures of Liquid Crystals, Verlag Chemie, Weinheim - New York 1978 oder H. Kelker, R. Hatz, Handbook of Liquid Crystals Verlag Chemie, Weinheim, Deerfield 1980).

Bevorzugte Verbindungen der Formel (I) sind solche, die 3 bis 25 aromatische Teilstrukturen enthalten, wobei der Rest -Ar- als eine Teilstruktur gewählt ist, unabhängig davon, ob er einkernig oder mehrkernig ist. Besonders bevorzugte Verbindungen der Formel (I) sind solche, die 4 bis 15 aromatische Teilstrukturen enthalten, wobei wiederum der Rest -Ar- als eine aromatische Teilstruktur gewählt ist, unabhängig davon ob er einkernig oder mehrkernig ist. Ganz besonders bevorzugte Verbindungen der Formel (I) sind solche, die 4 bis 10 aromatische Teilstrukturen im vorstehend definierten Sinne haben.

Als Verbindungen der Formel (I) seien beispielsweise die folgenden vier Gruppen von Verbindungen (I,A) bis (I,D) genannt:

(I,A) Flüssig kristalline Verbindungen der Formel (I) mit drei aromatischen Teilstrukturen, worin

m = Null, n = 1, o = 1 und p = Null sind,

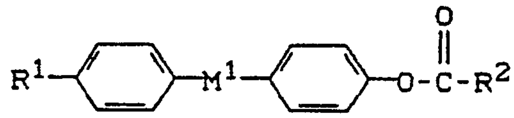 (Formel IA)

| Nr. | $R^1$ | $M^1$ | $R^2$ |
|---|---|---|---|
| 1 | HO | 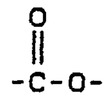 | 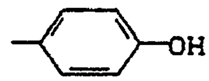 |
| 2 | HO | 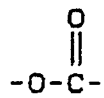 | 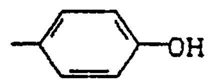 |

(I,B) Flüssigkristalline Verbindungen der Formel (I) mit vier aromatischen Teilstrukturen, worin wieder m = Null, n = 1, o = 1 und p = Null sind:

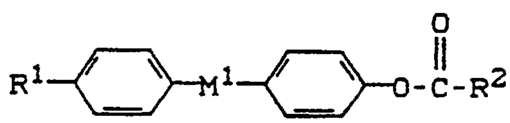 (Formel IB)

| Nr. | $R^1$ | $M^1$ | $R^2$ |
|---|---|---|---|
| 3 | 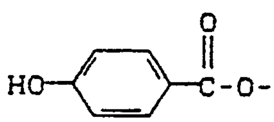 | 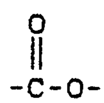 | 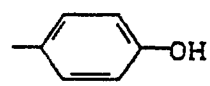 |
| 4 | 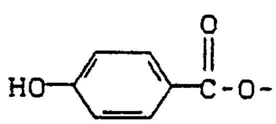 | 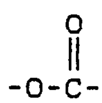 | 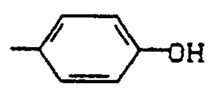 |
| 5 | 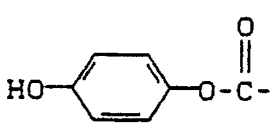 | 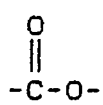 | 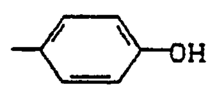 |

(I,C) Flüssigkristalline Verbindungen der Formel (I) mit fünf aromatischen Teilstrukturen, worin m = n = o = p = 1 sind.

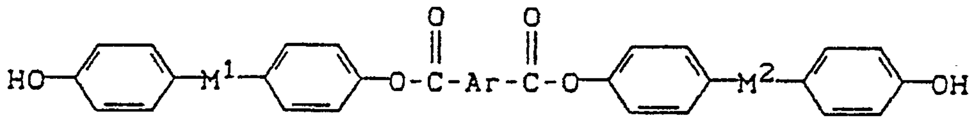

(Formel IC)

| Nr. | $M^1$ | $M^2$ | Ar |
|---|---|---|---|
| 6 | 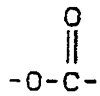 | 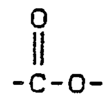 | 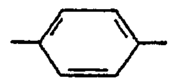 |
| 7 | " | " | 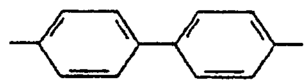 |
| 8 | 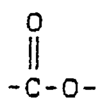 | 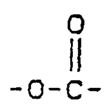 | 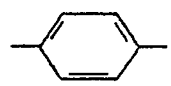 |
| 9 | " | " | 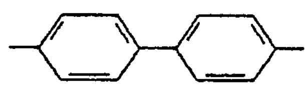 |

(I,D) Flüssigkristalline Verbindungen der Formel (I) mit sieben aromatischen Teilstrukturen, worin wieder m = n = o = p = 1 sind:

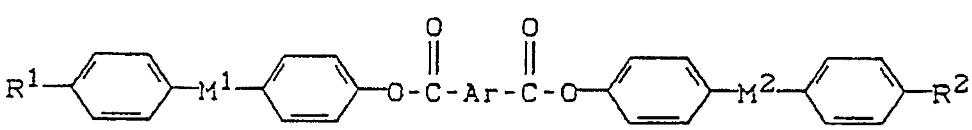

(Formel I D)

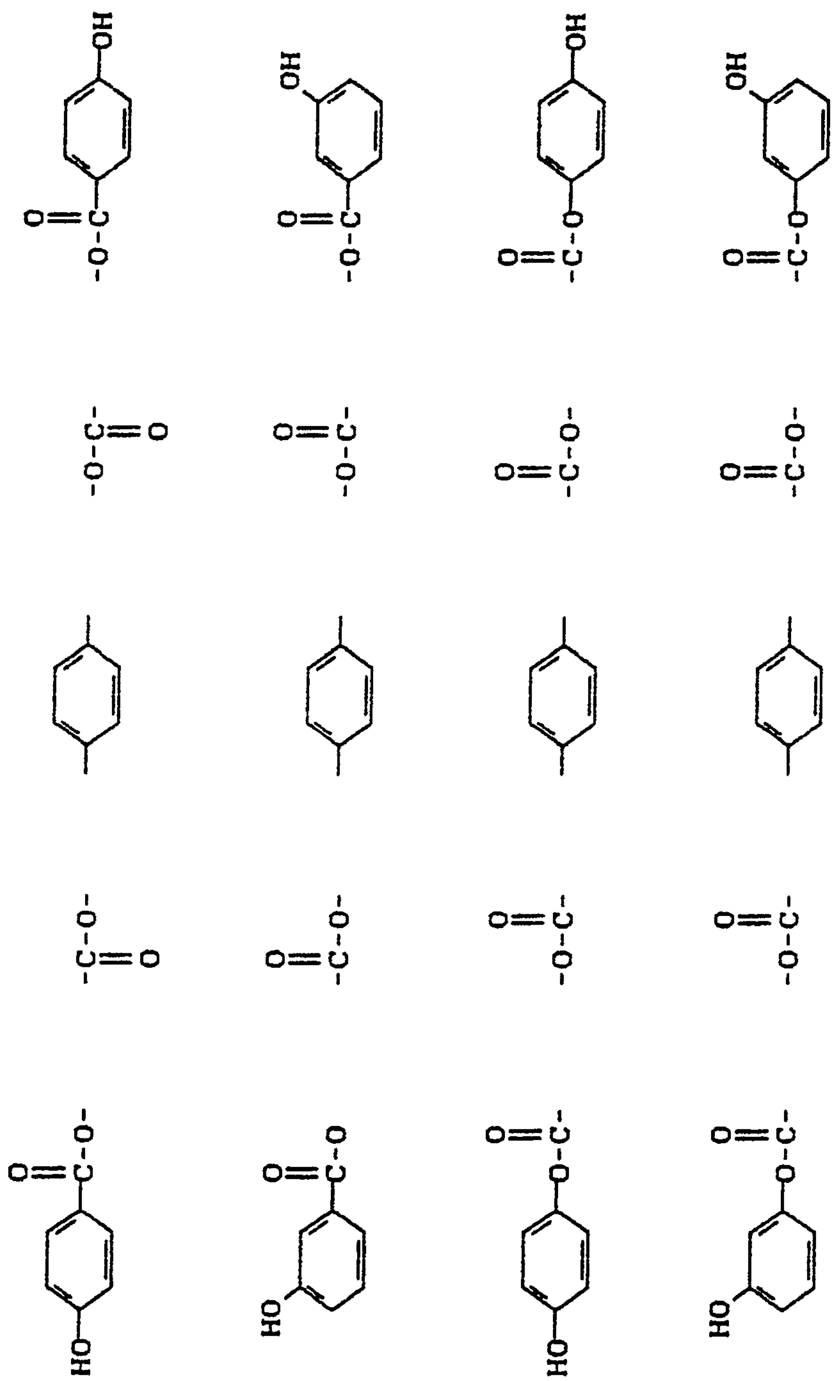

| Nr. | $R^1$ | $M^1$ | Ar | $M^2$ | $R^2$ |
|---|---|---|---|---|---|
| 10 | | | | | |
| 11 | | | | | |
| 12 | | | | | |
| 13 | | | | | |

Die erfindungsgemäßen niedermolekularen, flüssig kristallinen Additive (I), welche erfindungsgemäß eingesetzt werden können, können, gegebenenfalls unter Verwendung von Schutzgruppen, analog den Verbindungen der eigenen, bislang unveröffentlichten Patentanmeldung (P 3 824 365.2) hergestellt werden. Für ihre Herstellung wird im Beispielteil eine charakteristische Herstellungsvorschrift angegeben.

Die niedermolekularen, flüssig kristallinen Additive der Formel (I) werden bevorzugt zu 0,3-8 und besonders bevorzugt zu 0,5-5 Gew.-%, einzeln oder in einem beliebigen Gemisch, eingesetzt.

Verbindungen der Formel (II)

Als bivalente aromtische Reste ($Ar^a$) in der Formel (II) kommen solche in Betracht, die den aromatischen Hydroxycarbonsäuren der Formeln

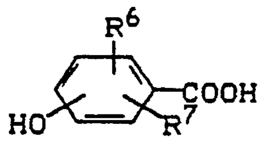

(XV)

und

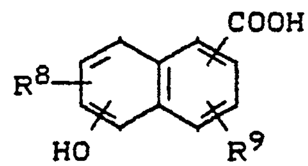

(XVI)

zugrundeliegen, worin

$R^6$ bis $R^9$ $C_1$-$C_4$-Alkyl (vorzugsweise Methyl, Ethyl), $C_1$-$C_4$-Alkoxy (vorzugsweise Methoxy, Ethoxy), $C_6$-$C_{10}$-Aryl oder -Aryloxy (vorzugsweise Phenyl, Phenyloxy, Naphthyl, Naphthyloxy, Biphenyl, Biphenyloxy, Tolyl, Tolyloxy), $C_7$-$C_{12}$-Alkylaryl (vorzugsweise Benzyl), Halogen (vorzugsweise Chlor und Brom) oder Wasserstoff bedeuten, und die Valenzen zwischen Kern und Hydroxylgruppe, sowie zwischen Kern und Carboxylgruppe einen Winkel von 45 bis 180° bilden.

Die Carboxylgruppen der Verbindungen der Formeln (XV) und (XVI) können in Ausnahmefällen auch über Alkylengruppen mit den aromatischen Resten verknüpft sein.

Bevorzugte aromatische Hydroxycarbonsäuren sind 4-Hydroxy-3-methylbenzoesäure, 4-Hydroxy-3-methoxybenzoesäure, 4-Hydroxybenzoesäure, 3-Hydroxybenzoesäure, 6-Hydroxy-2-naphthoesäure. Besonders bevorzugt sind 4-Hydroxybenzoesäure, 3-Hydroxybenzoesäure und 6-Hydroxy-2-naphthoesäure.

Die aromatischen Hydroxycarbonsäuren haben die OH-Gruppe vorzugsweise symmetrisch (1,4-Phenylen- oder 2,6-Naphthylen- oder 4,4'-Diphenyl-Positionen) zur Carboxylgruppe.

Als bivalente aromatische Reste ($Ar^b$) der Formel (II) kommen solche in Betracht, die den Diphenolen der Formel (IX)

HO-Z-OH (IX) zugrundeliegen,

worin

Z einen zweiwertigen ein- oder mehrkernigen aromatischen Rest mit 6 bis 30 C-Atomen bedeutet, wobei Z derart gebaut ist, daß die beiden OH-Gruppen direkt an je ein C-Atom eines aromatischen Systems gebunden sind und die beiden Valenzen einen Winkel von 45 bis 180° bilden.

Die aromatischen Reste können durch 1 bis 4 $C_1$-$C_4$-Alkyl-, $C_1$-$C_4$-Alkoxy-, Phenyl-, Phenoxy-, Benzyl- oder Halogenreste (vorzugsweise Chlor und Brom) substituiert sein und umfassen neben m/p-Phenylen-, 2,6- oder 1,5-Naphthylen- und 4,4'-Biphenylen-Resten auch durch Sauerstoff, Schwefel, Carbonyl, Sulfonyl, oder Azomethin, $C_1$-$C_{18}$-, vorzugsweise $C_1$-$C_4$-Alkylen oder -Alkyliden, (alkylsubstituiertes) Cyclohexylen oder -hexyliden oder $-O(CH_2)_nO-$mit $n = 2$ bis 4 verbundene Phenylenreste.

Bevorzugte Diphenole sind z.B. Hydrochinon, 4,4'-Dihydroxydiphenyl, 4,4'-Dihydroxydiphenylether, 4,4'-Dihydroxydiphenylethan, 4,4'-Dihydroxydiphenoxyethan, 3,5'-Dihydroxydiphenyl, 3,5'-Dihydroxydiphenylether, 1,5-Dihydroxynaphthalin, 2,6-Dihydroxynaphthalin, 1,4-Dihydroxynaphthalin, Methylhydrochinon, Phenylhydrochinon, 2,2'-Dimethyl-4,4'-dihydroxydiphenyl, 3,3',5,5'-Tetramethyl-4,4'-dihydroxydiphenyl, 1,2-(2-Chlor-4-hydroxyphenoxy)-ethan, 4-Methoxy-2,6-dihydroxynaphthalin, Resorcin, 3,4'-Dihydroxydiphenyl, 1,6-Dihydroxynaphthalin, 1,7-Dihydroxynaphthalin, 2,7-Dihydroxynaphthalin, 4-Chlorresorcin, 4-Phenylresorcin, 4-Ethoxyresorcin, 2,5-Dichlor-1,6-dihydroxynaphthalin und 4-Methoxy-2,7-dihydroxynaphthalin.

Besonders bevorzugte Diphenole sind Hydrochinon und 4,4'-Dihydroxydiphenyl.

Als bivalente Reste ($R^4$) der Formel (II) kommen solche in Betracht, die den Dicarbonsäuren der Formel (X)

$HOOC-R^4-COOH$ (X) zugrundeliegen,

worin

$R^4$ für $C_mH_{2m}$ mit $m = 0$-40, bevorzugt von 0-20 und von 30-38, oder auch für einen cycloaliphatischen Rest mit 5-15 C-Atomen steht, oder bevorzugt

einen bivalenten aromatischen Rest mit 6 bis 24 C-Atomen, vorzugsweise mit 6 bis 16 C-Atomen bedeutet,

wobei die beiden Valenzen einen Winkel von 45 bis 180° bilden. Die bivalenten aromatischen Reste können durch 1 bis 4 $C_1$-$C_4$-Alkyl-, $C_1$-$C_4$-Alkoxy-, Phenyl-, Phenoxy-, Benzyl- oder Halogenreste (vorzugsweise Chlor und Brom) substituiert sein und umfassen neben 1,4-Phenylen-Resten, 1,5- oder 2,6-Naphthylen-Resten und 4,4'- oder 3,5'-Biphenylen-Resten auch durch Sauerstoff, Schwefel, Carbonyl, Sulfonyl, $C_1$-$C_4$-Alkylen oder -Alkyliden, Cyclohexylen oder -hexyliden oder -$O(CH_2)_nO$- mit n = 1 bis 4 verbundene Phenylenreste, vorzugsweise in symmetrischer Substitution ("para-Stellung").

Bevorzugt sind folgende (cyclo)aliphatische Dicarbonsäuren: Oxalsäure, Bernsteinsäure, Adipinsäure, Korksäure, Azelainsäure, Trimethyladipinsäure, Sebacinsäure, Dodecandisäure, Dimerfettsäuren und 1-4-Cyclohexandicarbonsäure.

Besonders bevorzugt werden Oxalsäure, Adipinsäure, Sebazinsäure und Dimerfettsäuren eingesetzt.

Bevorzugte aromatische Dicarbonsäuren sind solche, die folgenden bivalenten Resten zugrundeliegen: 1,4-Phenylen-, 1,4-Naphthylen- oder 4,4'-Biphenylen-, worin die beiden Bindungen sich koaxial in entgegengesetzte Richtungen erstrecken, oder 1,5-Naphthylen-Reste, 2,6-Naphthylen-Reste oder 3,5'-Biphenylen-Reste, worin die beiden in entgegengesetzte Richtungen zeigenden Bindungen parallel zueinander verschoben sind, sowie 1,3-Phenylen-Reste, 1,3-, 1,6-, 1,7- oder 2,7-Naphthylen-Reste oder 3,4'-Biphenylen-Reste, worin die beiden Bindungen nicht an benachbarten Atomen lokalisiert sind und sich nicht koaxial oder parallel verschoben in entgegengesetzte Richtungen erstrecken.

Bevorzugte aromatische Dicarbonsäuren sind 1,4-Naphthalindicarbonsäure, 1,5-Naphthalindicarbonsäure, 2,6-Naphthalindicarbonsäure, Biphenyl-4,4'-dicarbonsäure, Bipenyl-3,3'-dicarbonsäure, Diphenoxyethan-4,4'-dicarbonsäure, Diphenylether-4,4'-dicarbonsäure, Methylterephthalsäure, Methoxyterephthalsäure, Chlorterephthalsäure, 4-Chlornaphthalin-2,7-dicarbonsäure, 2,6- oder 2,7-Naphthalindicarbonsäure, Biphenyl-3,4'-dicarbonsäure, 4-Methylisophthalsäure, 5-Methylisophthalsäure, Diphenylether-4,4'-dichlor-3,3'-dicarbonsäure, Iso- und Terephthalsäure.

Besonders bevorzugt sind Iso- und Terephthalsäure.

Es können sowohl ausschließlich aliphatische oder aromatische Dicarbonsäuren als auch Mischungen von beiden eingesetzt werden.

Als bivalente Reste ($R^5$) der Formel (II) kommen solche in Betracht, die den Aminoverbindungen der Formel (XI)

$H_2N$-$R^5$-Y (XI) zugrundeliegen,

worin

$R^5$ für $C_mH_{2m}$ mit m = 3 bis 40, bevorzugt 4 bis 12, und einen cycloaliphatischen Rest mit 5 bis 15 C-Atomen (5-6 Ring-C-Atomen), und

Y für OH, COOH oder $NH_2$ stehen, oder worin $R^5$

einen bivalenten aromatischen Rest mit 6 bis 24 C-Atomen, vorzugsweise mit 6 bis 16 C-Atomen bedeutet, wobei die beiden Valenzen einen Winkel von 45 bis 180° bilden. Die bivalenten aromatischen Reste können durch 1 bis 4 $C_1$-$C_4$-Alkyl-, $C_1$-$C_4$-Alkoxy-, Phenyl-, Phenoxy-, Benzyl- oder Halogenreste (vorzugsweise Chlor und Brom) substituiert sein und umfassen neben Phenylen-, Naphthylen- und Biphenylen-Resten auch durch Sauerstoff, Schwefel, Carbonyl, Sulfonyl, $C_1$-$C_4$-Alkylen oder -Alkyliden, Cyclohexylen oder -hexyliden oder -$O(CH_2)_nO$-mit n = 1 bis 4 verbundene Phenylenreste.

Anstelle der aliphatischen Aminocarbonsäuren können auch die entsprechenden Lactame, z.B. Caprolactam anstatt von C-Aminocapronsäure, eingesetzt werden.

Bevorzugte (cyclo)aliphatische Aminocarbonsäuren sind C-Aminocapronsäure bzw. Caprolactam, ω-Aminoundecansäure, ω-Aminododecansäure bzw. Laurinlactam, 4-Aminocyclohexyl-carbonsäure; bevorzugte aromatische Aminocarbonsäuren sind 4-Aminobenzoesäure oder 6-Amino-2-naphthoesäure.

Besonders bevorzugt sind ε-Aminocapronsäure bzw. Caprolactam, 4-Aminobenzoesäure und 6-Amino-2-naphthoesäure.

Bevorzugte Aminophenole sind z.B.:

3-Aminophenol, 4-Aminophenol, 3-Amino-2-methylphenol, 3-Amino-4-methylphenol, 5-Amino-1-naphthol, 6-Amino-1-naphthol, 5-Amino-2-naphthol, 7-Amino-2-naphthol, 8-Amino-2-naphthol, 6-Amino-2-naphthol und 4'-Amino-1-hydroxybiphenyl, besonders bevorzugt sind 4-Aminophenol und 3-Aminophenol, sowie 4'-Amino-1-hydroxy-biphenyl.

Bevorzugte Reste $R^3$ der Formel (II) sind, je nach Verknüpfung mit den anderen Bausteinen, die Reste (XVII) bis (XXIV).

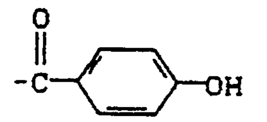

(XVII)

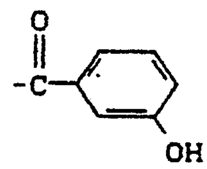

(XVIII)

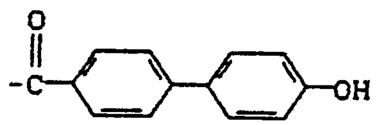

(XIX)

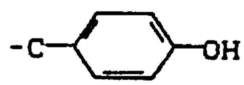

(XX)

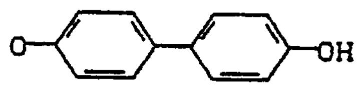

(XXI)

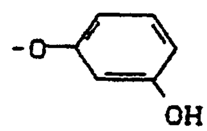

(XXII)

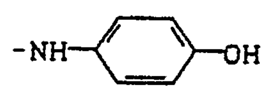

(XXIII)

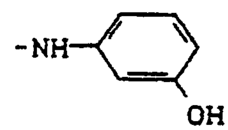

(XXIV)

Bevorzugte Oligomere der Formel (II) sind solche, die durchschnittlich 3 bis 25, besonders bevorzugt 4 bis 20, insbesondere 4 bis 15 aromatische Teilstrukturen enthalten, wobei eine aromatische Teilstruktur wie unter Formel (I) definiert ist.

Die Herstellung der obengenannten Verbindungen der Formel (II) erfolgt nach bekannten Schmelz- und Umesterungsverfahren, die auch zur Herstellung von thermotropen LC-Polymeren herangezogen werden (Direktveresterungsverfahren [EP-A 00 88 546], Acetatverfahren [EP-A 01 02 719, 134 204], Schmelzumesterung von Carbonsäurephenylestern mit Diphenolen (Diphenylesterverfahren) [EP-A 072 540, 070 539, 024 499, 45 499, DE-OS 20 25 971, EP 070 539 und EP 132 637]). Dabei wird die Kettenlänge durch den molaren Überschuß hydroxylgruppenhaltiger Bausteine im Vergleich zu den Dicarbonsäuren festgelegt.

Gegebenenfalls können zusätzlich noch übliche Kettenabbrecher, z.B. Monocarbonsäuren oder Monophenole, mitverwendet werden.

Die erfindungsgemäßen Verbindungen der Formel (II) werden besonders bevorzugt nach dem Acetat- und/oder nach dem Diphenylesterverfahren hergestellt. Es sind oligomere Gemische mit mittleren Molekulargewichten ($\bar{M}n$) ≦4000, vorzugsweise ≦2500, welche sowohl ihre genannten Struktureinheiten wie auch ihr Molekulargewicht in statistischer Verteilung besitzen können und flüssig kristalline Eigenschaften aufweisen. Sie werden als LC-Additive 2) in den angegebenen Mengen zur Herstellung der neuen Polyamid-Formmassen und -Legierungen eingesetzt, wobei die flüssigkristallinen, von aliphatischen Bausteinen freien, oligomeren Verbindungen der Formel (II) bevorzugt sind, insbesondere in statistischer Verteilung der Komponenten in (II).

Die niedermolekularen, flüssigkristallinen Additive (II) werden zu 0,1-15, bevorzugt 0,3-8 und besonders bevorzugt 0,5-5 Gew.-% bei der Herstellung der erfindungsgemäßen Polyamid-Formmassen eingesetzt. Sie können einzeln oder in einem beliebigen Gemisch eingesetzt werden.

Bevorzugt einzusetzende LC-Additive 2) sind die statistisch aufgebauten Verbindungen der Formel (II).

Gegenstand der Erfindung sind auch die neuen flüssigkristallinen Ester- und/oder Esteramid-Oligomeren

der Formel (II), wobei die Symbole die bereits aufgeführten Bedeutungen haben.

Gegenstände der Erfindung sind weiterhin die Verwendung der erfindungsgemäßen Polyamid-Formmassen zur Herstellung von Formkörpern, Folien, Fasern, Verbundwerkstoffen und anderen Gegenständen sowie Formkörper, Folien, Fasern, Verbundwerkstoffe und andere Gegenstände aus den neuen Polyamid-Formmassen.

Polyamide:

Polyamide gemäß 1), welche erfindungsgemäß eingesetzt werden können, umfassen teilkristalline und amorphe Polyamide. Sie können einzeln oder in einem beliebigen Gemisch eingesetzt werden. Beispiele für Polyamide im Sinne der Erfindung sind PA 6, 11, 12, 46, 66, 67, 68, 69, 610, 1012, 612, 1010, 1212, 6/66-Copolyamide, 6/12-Copolyamide, 6/11-Copolyamide, 66/11-Copolyamide, 66/12-Copolyamide, 6/610-Copolyamide, 66/610-Copolyamide, 6/66/610-Terpolyamide, 6I6-Copolyamide, 6T/6- bzw. 6T6-Copolyamide, 6IT-Copolyamide, PA 6I, Copolyamide aus 1,4-Cyclohexandicarbonsäure, 2,2,4- und 2,4,4-Trimethylhexamethylendiamin, Copolyamide aus Terephthalsäure, 2,2,4- und 2,4,4- Trimethylhexamethylendiamin, Copolyamide aus Isophthalsäure, Laurinlactam und 3,5'-Dimethyl-4,4'-diamino-dicyclohexylmethan, Copolyamide aus Isophthalsäure, Azelainsäure und/oder Sebacinsäure und 4,4'-Diaminodicyclohexylmethan (bzw. Isomerengemische), Copolyamide aus Caprolactam, Isophthalsäure (+ ggf. Terephthalsäure) und 4,4'-Diaminodicyclohexylmethan (bzw. Isomerengemische), Copolyamide aus Caprolactam, Isophthalsäure (+ ggf. Terephthalsäure) und Isophorondiamin, Copolyamide aus Isophthalsäure (+ ggf. Terephthalsäure) und/oder anderen aromatischen oder aliphatischen Dicarbonsäuren, Hexamethylendiamin und/oder ggf. alkyl-substituierten Hexamethylendiaminen und in Nachbarschaft zu den Aminogruppen alkyl-substituierten 4,4'-Diaminodicyclohexylaminen, sowie andere Copolyamide oder Gemische verschiedener Polyamide aus Komponenten, wie sie üblicherweise für Polyamide bekannt sind. Auch PolyamidBlockpolymere wie Polyether- und Polyetheresteramide können eingesetzt werden.

Auch Polyamide, welche aus Dicarbonsäuren und Diisocyanaten herstellbar sind, sind geeignet (z.B. Copolyamide aus Adipinsäure, Azelainsäure und 4,4'-Diphenylmethandiisocyanat und Copolyamide aus Terephthalsäure, Azelainsäure, 4,4'-Diphenylmethandiisocyanat und 2,4-(2,6)-Toluylendiisocyanat).

Bevorzugte Polyamide, einzeln oder in einem beliebigen Gemisch, sind PA 6, 46, 66, 610, 1012, 11, 12, 1212, 6I6, 6I, 6T/6, 6T6, 6/66-Copolyamide, Copolyamide aus Caprolactam, Isophthalsäure und Isophorondiamin, Copolyamide aus Isophthalsäure (+ ggf. Terephthalsäsure), Azelainsäure und 4,4'-Diaminodicyclohexylmethan (bzw. Isomerengemische) und Copolyamide aus Isophthalsäure (+ ggf. Terephthalsäure), Caprolactam und 4,4'-Diaminodicyclohexylmethan (bzw. Isomerengemische).

Ganz besonders bevorzugt werden Polyamid 6 und 66 eingesetzt.

Legierungspartner

Als Polymere Legierungspartner (einer der möglichen Zusatzstoffe gemäß 3)) zur Herstellung der neuen, leichtfließenden Polyamid-Legierungen, die immer neben den Polyamiden mindestens einen polymeren Legierungspartner enthalten, kommen, allein oder im Gemisch, z.B. in Betracht: Dienkautschuke, Acrylat-Kautschuke, Polyethylene, Polypropylene, Ethylen-Propylen-Copolymere, Ethylen-1-Buten-Copolymere, Ethylen-Propylen-Dien-Terpolymere, Ethylen-und/oder Propylen-Acrylsäure/Acrylester-Copolymere, Ethylen-Vinylacetat-Copolymere, Polyoctenylene, Polystyrole, ($\alpha$-Methyl)Stryrol-(Meth)acrylnitril-copolymere, (Meth)Acrylnitril-Butadien-($\alpha$-Methyl)Styrol-Polymere (ABS), schlagzähe Polystyrole, Polycarbonate, aromatische Polyester(carbonate), Polyester wie z.B. Polyethylenterephthalat, Polysulfone, Polyphenylenoxide, Polyetherketone, Polyarylensulfide, Polyetheretherketone, Polyamidimide, Polyethersulfone, Polyetherimide, Polyesterimide und Polyimide, wie sie im Stand der Technik als Legierungspartner bzw. Modifikatoren bekannt sind.

Die polymeren Legierungspartner sollten, gegebenenfalls zumindest teilweise, chemisch so modifiziert sein, daß eine partielle Ankopplung der beiden Phasen, z.B. über Carbonsäuregruppen, erfolgt. Dies kann z.B. dadurch geschehen, daß ein Copolymer aus Ethylen und/oder Propylen und geringen Mengen Acrylsäure, oder ein mit geringen Mengen Maleinsäureanhydrid gepfropftes Ethylen-Propylen-Dien-Polymer oder ein mit geringen Mengen Maleinsäureanhydrid gepfropftes Polyphenylenoxid allein oder im Gemisch mit unmodifizierten Legierungspartnern eingesetzt werden. Ankopplung kann auch über Ester- oder Epoxidgruppen erfolgen. Es kann weiter z.B. dadurch geschehen, daß geeignete niedermolekulare oder polymere Verträglichkeitsvermittler anwesend sind, z.B. kann in Legierungen mit ABS ein Acrylnitril-Styrol-Acrylsäure-Terpolymer als Verträglichkeitsvermittler eingesetzt werden. Die Legierungspartner können auch reaktive Endgruppen aufweisen, welche mit dem Polyamid reagieren können, z.B. Amino-oder Carboxyl-terminierte

Polydien-Kautschuke.

Die Kautschuke können auch in einer Kern-Mantel-Struktur gepfropft sein.

Es können erfindungsgemäß auch Gemische aus Polyamiden mit mehr als einem Polymeren bzw. Legierungspartner erhalten werden, z.B. Legierungen aus Polyamid 66, Polyphenylenoxid und schlagzähem Polystyrol, oder Legierungen aus Polyamid 66, aromatischen Polyestern und einem Schlagzähmodifikator; bei solchen Gemischen sollte der Polyamid-Anteil nicht unter 30 Gew.-%, vorzugsweise nicht unter 50 Gew.-%, bezogen auf die fertige Mischung, betragen.

Die polymeren Legierungspartner können bereits vorab mit Polyamiden oder Mischungen von Polyamiden zu Polyamid-Legierungen (vor)vermischt werden, oder werden bevorzugt als einer der Zusatzstoffe entsprechend 3) eingesetzt. Dabei werden insbesondere mindestens ein Polyamid, mindestens ein polymerer Legierungspartner, mindestens ein LC-Additiv 2) gemäß Formel (I) und/oder (II) sowie gegebenenfalls weitere, übliche Zusatzstoffe 3) wie Glasfasern oder auch Stabilisatoren, in der Schmelze (vorzugsweise in Extrudern) vermischt.

Im Fall von Polyamid-Legierungen ist also neben den Polyamiden gemäß 1) und den LC-Fließverbesserern gemäß 2) immer mindestens ein Zusatzstoff gemäß 3), nämlich der polymere Legierungspartner, anwesend in der fertigen Polyamid-Legierung.

Gegenstand der Erfindung ist auch die Verwendung der flüssigkristallinen, niedermolekularen Ester und/oder Esteramide (I) und/oder der oligomeren Ester und/oder Esteramide (II) als LC-Fließverbesserungsmittel-Zusatz 2) in Mengen von 0,1 bis 15 Gew.-%. (oder den obengenannten bevorzugten Mengen) bei der Herstellung von Polyamid-Formmassen. Weiterhin ist Erfindungsgegenstand ein Verfahren zur Herstellung der neuen leichtfließenden Polyamid-Formmassen durch Vermischen mindestens eines Polyamids 1), mindestens eines LC-Fließverbesserers (I) und/oder (II) 2) sowie gegebenenfalls der Zusatzstoffe gemäß 3) in der Schmelze, wobei gegebenenfalls die Zusatzstoffe gemäß 3) auch in einem gesonderten Schritt vorher in das Polyamid 1) eingebracht worden sein können, so daß dann unter 1) ein entsprechend modifiziertes Polyamid eingesetzt wird. Bevorzugt geschieht die Herstellung der neuen Polyamid-Formmassen in Extrudern oder Knetern.

Der Zusatz von flüssig-kristallinen Polymeren zu Thermoplasten ist bekannt (siehe beispielsweise EP-OS 0 030 417). Ein Nachteil ist jedoch im allgemeinen die begrenzte Verträglichkeit der Thermoplasten mit den flüssig-kristallinen Polymeren.

Unschmelzbare, whiskerartige Poly-(p-oxybenzoat)-kristalle werden in der US-Patentschrift 4 673 724 als Verstärkungskomponente beschrieben. Diese sind wegen ihrer sehr hohen Schmelz- bzw. Erweichungspunkte jedoch sehr schwer herstellbar und mit anderen Polymeren besonders unverträglich.

In situ erzeugte Mischungen aus flüssig-kristallinen Polymeren und amorphen Thermoplasten sind ebenfalls bekannt. (Siehe beispielsweise G. Kiss, Polymer Engineering & Science, 27, Seiten 410-423 (1987). Derartige Mischungen haben jedoch den Nachteil, daß die erreichbaren Verstärkungseffekte in starkem Maß von den Mischungsbedingungen abhängen und die Reproduzierbarkeit der Verarbeitungsbedingungen oft problematisch einzuhalten sind.

Hinzu kommt, daß bei der Herstellung derartiger Composites die Mischungspartner sehr unterschiedliche Schmelzviskositäten unter Verarbeitungsbedingungen besitzen, so daß zur Herstellung von solchen Composites nur eine beschränkte Zahl von geeigneten Polymeren bereitsteht.

Als Verarbeitungshilfsmittel für konventionelle Thermoplaste wie aromatische Polyester oder thermoplastisch schwer verarbeitbare, flüssigkristalline Polymere werden chemisch reaktive, flüssig-kristalline, aromatische Ester beschrieben, die durch Umesterung bei höherer Temperatur in die Polymerkette einkondensieren. (Siehe US-Patent 4 650 836). Der Einbau der aromatischen, flüssig-kristallinen Ester in die Polymerkette führt jedoch zu einer chemischen Veränderung der Thermoplasten. Dies hat den Nachteil, daß in Abhängigkeit der Verarbeitungsbedingungen unterschiedliche Einbauverhältnisse und damit schlecht reproduzierbare Eigenschaften resultieren. Der überraschende Effekt der erfindungsgemäßen Mischung ist bei solchen Copolymerisaten jedoch nicht mehr vorhanden.

Die Herstellung der erfindungsgemäßen, leichtfließenden Formmassen kann nach allen in der Thermoplast-Verarbeitung zur Abmischung üblichen Verfahren geschehen, wobei bevorzugt solche Bedingungen (Verweilzeiten/Temperaturen) eingehalten werden sollten, daß keine bzw. nur eine geringe Umesterung erfolgt und bevorzugt die flüssigkristallinen Additive im wesentlichen unreagiert vorliegen.

Zur Herstellung der erfindungsgemäßen, LC-Fließverbesserer 2) enthaltenden, leicht fließenden Polyamid-Formmassen können beispielsweise die Polyamidkomponente(n) 1), die LC-Fließverbesserer (I) und/oder (II) 2) sowie die gegebenenfalls einzusetzenden Zusatzstoffe 3) (z.B. Legierungskomponenten, Verstärkungsstoffe, Stabilisatoren u.a.) in der Schmelze miteinander vermischt werden. Vorzugsweise werden die erfindungsgemäßen Polyamid-Formmassen durch Mischen aller Ausgangskomponenten in üblichen Schneckenmaschinen hergestellt. Der flüssig kristalline LC-Fließverbesserer 2) kann dabei anfangs

mit der Polyamid-Komponente 1) schon gemeinsam in den Extruder eindosiert oder erst zu einem späteren Zeitpunkt während der Extrusion den zu extrudierenden Massen zugesetzt werden.

Die LC-Fließverbesserer 2) (I) und/oder II) können also zu jedem Zeitpunkt bei der Herstellung der erfindungsgemäßen Formmassen eingesetzt werden. Auch ein Zusatz unmittelbar vor der Verarbeitung von Polyamid-Formmassen ist möglich.

Die LC-Additive 2) können als solche oder auch in Form von Konzentraten in einem (vorzugsweise niedrigerschmelzenden) Polymeren zugegeben werden.

Die Polyamide 1) können die Zusatzstoffe gemäß 3) bereits vorher eingemischt enthalten. Bevorzugt werden aber diese Komponenten 3) zusammen mit den flüssigkristallinen Komponenten 2) und den Polyamiden 1) in einem Arbeitsgang vermischt.

Die erfindungsgemäß mit LC-Fließverbesserern 2) modifizierten, leicht fließenden PA-Formmassen können noch weitere, übliche Zusatzstoffe 3) in der Menge von 0,001 bis 150 Gew.-%, bevorzugt 0,01 bis 100 Gew.-%, bezogen auf die Summe der Gewichte von 1) und 2), enthalten. Hier kommen Verstärkungsmaterialien (Glasfasern, Aramidfasern, Kohlenstoffasern, Glaskugeln, $SiO_2$, Kreide, Talkum, Kaolin, Glimmer u.a.), Weichmacher, Antioxidantien, Pigmente, Farbstoffe, Bewitterungsstabilisatoren (bzw. Stabilisatorkombinationen), Gleitmittel, Fließhilfsmittel, Entformungsmittel, Nukleierungsmittel, wie z.B. Polyarylensulfide, die Wasseraufnahme verringernde Zusätze (z.B. Monophenole, Bisphenole, (Alkyl)phenol-Formaldehyd-Kondensate u.a.), Flammschutzmittel und andere in Frage, wie sie für entsprechende Polyamidmassen grundsätzlich bekannt und vorgeschlagen sind. Die Zusatzstoffe gemäß 3) können unabhängig voneinander für sich oder in Form eines Konzentrats eingesetzt werden. Sie können aber auch schon ganz oder teilweise in den Polyamiden 1) enthalten sein.

Die erfindungsgemäßen Polyamid-Formmassen können z.B. nach den Verfahren des Spritzgießens und der Extrusion zu Formkörpern und anderen Gegenständen verarbeitet werden. Sie eignen sich aufgrund ihrer drastisch verbesserten Fließfähigkeit insbesondere für großflächige und komplizierte Formteile, die sonst nur mit hohem technischen Aufwand bei der Verarbeitung, oft mit Oberflächenstörungen, oder häufig auch gar nicht, hergestellt werden können.

Vorzugsweise eignen sie sich für Anwendungen im Automobilbau.

Die erfindungsgemäßen PA-Formmassen zeichnen sich schon bei einer geringen Einsatzmenge an LC-Additiv 2) durch eine drastisch erhöhte Fließfähigkeit sowie gegebenenfalls auch durch verbesserte mechanische Eigenschaften und Wärmebeständigkeiten aus. Sie können sich weiterhin durch verringerte Wasseraufnahme sowie höhere Kristallisationsgeschwindigkeit und Kristallisationsgrad auszeichnen. Sie sind somit eine wertvolle Bereicherung des Standes der Technik.

Gegenstand der Erfindung ist auch die Verwendung der erfindungsgemäßen Additive (I) und (II) als Additive für andere Thermoplaste, z.B. PPS, thermoplastische Polyurethane, ABS, PVC, Polysulfone, Polyethylen, Polypropylen, u.a.m.

Die folgenden Beispiele dienen zur Erläuterung der Erfindung, ohne sie darauf einzuschränken.

Beispiel 1

4-(4-Hydroxybenzoyloxy)-benzoesäure-4-(4-Hydroxybenzoyloxy)-phenylester

1. Stufe: 4-Hydroxyphenyl-4-hydroxybenzoat

138,1 g p-Hydroxybenzoesäure und 110,1 g Hydrochinon werden zusammen mit 2 g Borsäure und 2,5 g Schwefelsäure in 900 ml Xylol suspendiert und bis zum Ende der Wasserabspaltung unter Rückfluß erhitzt. Das Produkt wird abgesaugt, getrocknet und mit verdünnter Na-Bicarbonat-Lösung gewaschen, erneut getrocknet und in 500 ml Aceton gelöst. Die Lösung wird heiß vom Ungelösten abfiltriert und das Produkt aus Wasser gefällt, abgesaugt und getrocknet.
Ausbeute: 182 g
Schmp.: 245-247 °C

2. Stufe: 4-(4-Carbobenzoxybenzoyloxy)-benzoesäure-4-(4-carbobenzoxybenzoyloxy)-phenylester

34,5 g 4-Hydroxyphenyl-4-hydroxybenzoat werden mit 24,9 g Pyridin in 300 ml Methylenchlorid suspendiert und bei 0 °C innerhalb von 3h mit 35,4 g 4-Carbobenzoxybenzoylchlorid versetzt. Nach 2h wird das Lösungsmittel im Vakuum abdestilliert und der Rückstand mit 140 ml 5 %iger Salzsäure aufgefüllt. Das unlösliche Produkt wurde abgesaugt, mit Wasser gewaschen und getrocknet.
Ausbeute: 50,3 g (91 % der Theorie)

3. Stufe: 4-(4-Hydroxybenzoyloxy)-benzoesäure-4-(4-Hydroxybenzoyloxy)-phenylester

Das Produkt aus Stufe 2 wird in 150 ml Ethylacetat suspendiert, mit 0,2 Palladiumkohle versetzt und bei 2 atm Druck hydriert (2h). Der Katalysator wird abfiltriert und das Lösungsmittel unter Vakuum abdestilliert.

Der farblose Feststoff ist in der Regel DC-rein, kann zur weiteren Reinigung aus DMF umkristallisiert werden. Ausbeute: 38 g

Im Bereich von 165-280 °C kann unter dem Polarisationsmikroskop eine LC-Phase festgestellt werden.

Beispiel 2

Statistisch aufgebaute LC-Ester

In einem Reaktionsgefäß mit Rührer, Kolonne und Destillierbrücke werden
207,2 g p-Hydroxybenzoesäure
165,2 g Hydrochinon
124,6 g Terephthalsäure
0,08 g Magnesium und
306,6 g Acetanhydrid
eingewogen.

Bei einer Innentemperatur von 160 ° C begann die Abspaltung der Essigsäure. Mit nachlassender Destillationsgeschwindigkeit wurde die Reaktionstemperatur langsam auf 250 ° C erhöht.

Nach Beendigung dar Destillation wurde der Druck im Verlauf von 30 Min. bis auf 1 mbar gesenkt. Während der Vakuumphase wurden restliche Mengen an Essigsäure im Verlauf von 2h abgespalten.

Das Produkt wurde in eine Weißblechdose abgefüllt und nach dem Erkalten zerkleinert.

Ausbeute: 450 g.

Im Bereich zwischen 180 ° C-300 ° C wurde mittels Polarisationsmikroskopie eine flüssigkristalline Phase festgestellt.

Beispiel 3

Analog zur Vorschrift in Beispiel 2 werden
207,2 g p-Hydroxybenzoesäure
165,2 g Hydrochinon
124,6 g Isophthalsäure
0,08 g Magnesium und
306,6 g Acetanhydrid
eingewogen und umgesetzt.

Ausbeute: 490 g.

Im Bereich von 170 und 320 ° C wurde mittels Polarisationsmikroskopie eine flüssigkristalline Phase festgestellt.

Beispiel 4

Ein Polyamid-6 Granulat mit einer rel. Lösungsviskosität von 2,9 in m-Kresol wurde mit steigenden Mengen der Additive aus den Beispielen 1+2 bei T = 290 °C auf einem Eiswellenextruder compoundiert und das Granulat zu Probekörpern (80x10x4 mm) verarbeitet, an denen der Biegeversuch (nach DIN 53452) durchgeführt wurde.

Außerdem wurde die Wasseraufnahme (15 Tage) bestimmt.

Die Meßwerte sind in Tabelle 1 zusammengefaßt.

Das Vergleichsbeispiel zeigt anschaulich die Verbesserung der Fließfähigkeit durch die LC-Additive (Reduzierung der Schmelzviskosität).

Weitere Eigenschaftsverbesserungen sind die Erhöhung von Steifigkeit bei hoher Randfaserdehnung und die Reduzierung der Wasseraufnahme.

**Tabelle 1** (Beispiel 4)

Compoundierung eines Polyamids-6 mit einer rel. Lösungsviskosität von ca. 2,9 in m-Kresol

| Additiv aus Beispiel | Konz. [%] | $\eta_{rel}$ | $\eta$ (Schmelze) $T = 270^0 C$ $\gamma = 100 s^{-1}$ | $H_2O$-Aufnahme [%] | Biege-E-Modul [mPa] | Biegefestigkeit [mPa] | Randfaserdehnung [%] |
|---|---|---|---|---|---|---|---|
| Vergleichsbeispiel | 0 | 2,85 | 115 | 8,6 | 2698 | 112 | 5,69 |
| 2 | 1 | 2,8 | 100 | 8,4 | 2745 | 112 | 5,87 |
| | 3 | 2,75 | 90 | 7,5 | 2715 | 112 | 5,82 |
| | 5 | 2,75 | 40 | 7,2 | 2812 | 114 | 5,88 |
| 1 | 1 | 2,85 | 100 | 8,2 | 2760 | 112 | 5,70 |
| | 3 | 2,80 | 85 | 7,1 | 2780 | 114 | 5,80 |
| | 5 | 2,75 | 55 | 7,0 | 2850 | 114 | 5,75 |

Beispiel 5

Ein PA 6,6 Granulat mit einer rel. Lösungsviskosität von 2,9 im n-Kresol wurde mit dem Additiv aus Beispiel 1 und 2 bei T = 290° C auf einem Einwellenextruder compoundiert. Das Granulat wurde an

Probekörpern (80x10x4 mm) verarbeitet, an denen der Biegeversuch (nach DIN 53452) durchgeführt wird.

Außerdem wurde die Wasseraufnahme (15 Tage) bestimmt.

Die Meßwerte sind in Tabelle 2 zusammengefaßt.

Das Vergleichsbeispiel demonstriert die Verbesserung der Fließfähigkeit (Reduzierung der Schmelzviskosität). Weitere Eigenschaftsverbesserungen sind die verminderte Wasseraufnahme und die Steifigkeitserhöhung bei hoher Randfaserdehnung.

Tabelle 2 (Beispiel 5)

Compoundierung eines Polyamids 6,6 mit einer rel. Lösungsviskosität von ca. 2,9 in m-Kresol

| Additiv aus Beispiel | Konz. [%] | $\eta_{rel}$ | $\eta$ (Schmelze) $T = 270^0 C$ $\gamma = 100 s^{-1}$ | $H_2O$-Aufnahme [%] | Biege-E-Modul [mPa] | Biege-festigkeit [mPa] | Randfaser-dehnung [%] |
|---|---|---|---|---|---|---|---|
| Vergleichs-beispiel | 0 | 2,85 | 200 | 7,65 | 2856 | 118 | 5,83 |
| 1 | 1 | 2,85 | 175 | 7,20 | 2900 | 120 | 6,00 |
| | 3 | 2,85 | 135 | 6,20 | 2940 | 124 | 6,15 |
| | 5 | 2,80 | 80 | 6,0 | 3060 | 124 | 5,80 |
| 2 | 1 | 2,85 | 150 | 7,35 | 2956 | 124 | 6,24 |
| | 3 | 2,80 | 85 | 6,55 | 3063 | 126 | 6,01 |
| | 5 | 2,82 | 40 | 5,80 | 3107 | 115 | 4,21 |

## Patentansprüche

1. Leichtfließende Polyamid-Formmassen, dadurch gekennzeichnet, daß sie durch Vermischen von
   1) 85-99,9 Gew.-% an sich bekannter Polyamide mit

2) 0,1 bis 15 Gew.-%, bevorzugt 0,3 bis 8 Gew.-%, besonders bevorzugt 0,5 bis 5 Gew.-% hydroxylgruppenhaltiger, niedermolekularer, flüssig kristalliner Ester und/oder Esteramide ("LC-Fließverbesserer") des allgemeinen Strukturtyps (I)

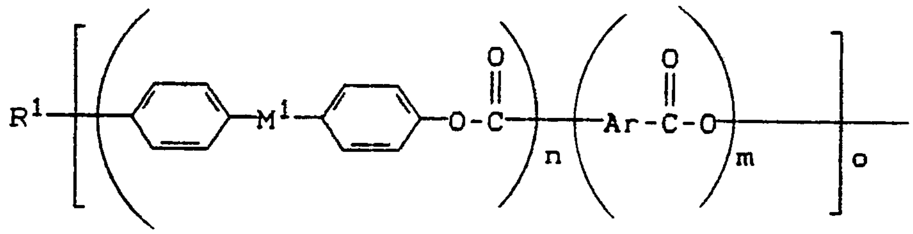

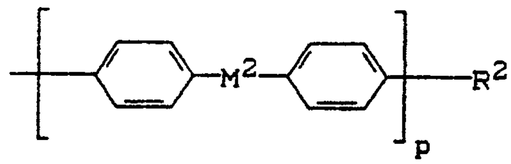

(Formel I)

worin

-Ar ein aromatischer Rest mit 6-20 C-Atomen ist, der einkernig oder mehrkernig sein kann, wobei der mehrkernige Rest über eine Bindung verknüpft oder annelliert sein kann,

worin

$R^1$ und $R^2$ gleich oder verschieden sein können und für einen Rest der Formeln (III) - (XI) stehen,

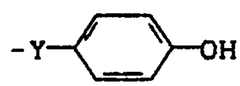

(III)

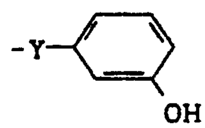

(IV)

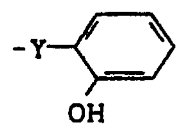

(V)

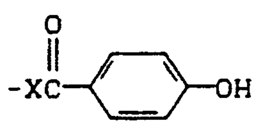

(VI)

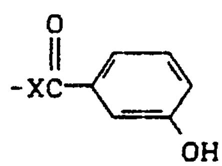

(VII)

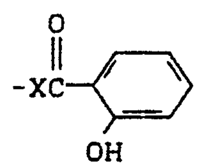

(VIII)

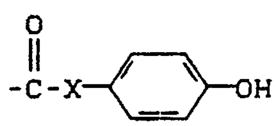

(IX)

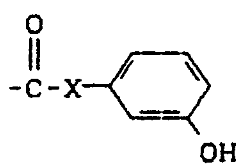

(X)

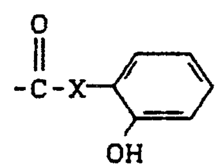

(XI)

(Reste (III)-(XI) entsprechend ihrer Abfolge),

Y eine chemische Bindung oder einen zweiwertigen Kohlenwasserstoffrest mit 1-10 C-Atomen oder auch eine $-SO_2$-Gruppe, bevorzugt eine chemische Bindung oder einen $-C(CH_3)_2$-Rest bedeutet,

X für O oder NH steht,

und wobei die aromatischen Ringe in den Formeln (III) - (XI) auch mit beispielsweise Halogenatomen oder Alkylgruppen substituiert sein können,

worin $-M^1$ und $-M^2$ gleich oder verschieden sind und zweibindige Reste der Formeln I.1) bis I.11)

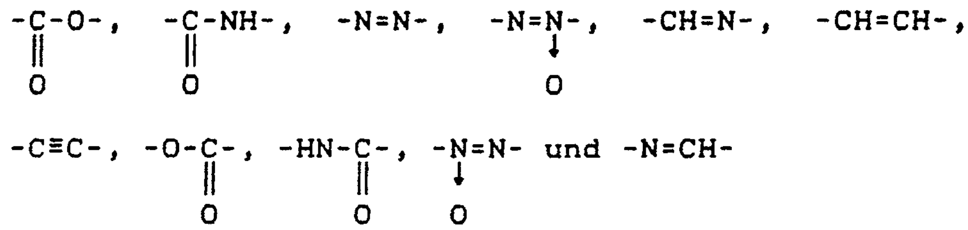

(Formeln (I.1) - (I.11), entsprechend ihrer Aufeinanderfolge) sein können,
und worin

- m Null, 1 oder 2,
- n 1 oder 2,
- o 1, 2, 3 oder 4 und
- p Null oder 1 sind,

und/oder
flüssig kristalliner Ester- und/oder Esteramid-Oligomerer des Strukturtyps der Formel (II) mit statistischer Verteilung der Struktureinheiten

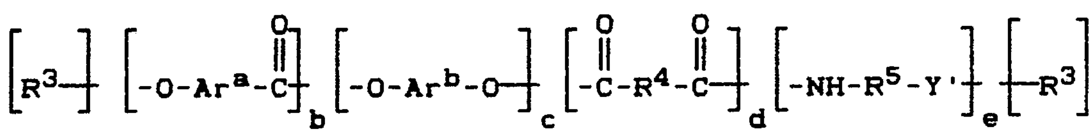

**Formel (II)**

worin

Ar[a] ein bivalenter, gegebenenfalls substituierter (Substituenten wie in $R^3$), ein- oder mehrkerniger aromatischer Rest mit 6-24 C-Atomen ist, wobei der mehrkernige Rest direkt verknüpft oder auch anelliert sein kann und wobei die Carboxylgruppe auch über einen aliphatischen Rest mit dem aromatischen Ring verknüpft sein kann,

Ar[b] einen bivalenten, gegebenenfalls substituierten, ein- oder mehrkernigen aromatischen Rest mit 6-30 C-Atomen darstellt, wobei der mehrkernige Rest unterschiedlich verknüpft sein kann,

$R^4$ bzw. $R^5$ für einen Alkylrest mit 0-40 ($R^4$) bzw. 3-40 ($R^5$) C-Atomen stehen oder beide auch für einen cycloaliphatischen Rest mit 5-15 C-Atomen stehen können oder die Bedeutung eines gegebenenfalls substituierten bivalenten, ein-oder mehrkernigen aromatischen Restes mit 6-24 C-Atomen haben, wobei der mehrkernige Rest unterschiedlich über eine Bindung oder auch anelliert verknüpft sein kann,

Y' für -O-,

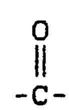

oder -NH- steht,

b = Null bis 10, vorzugsweise bis 8, insbesondere bis 4,

c = 1 bis 9, vorzugsweise bis 7, insbesondere 1 bis 4,

d = Null bis 9, vorzugsweise bis 7, insbesondere bis 3,

e = Null bis 3, vorzugsweise bis 2, ist und

worin

$R_3$ je nach Verknüpfung mit den anderen Bausteinen für die Reste der Formeln (XII) - (XIV),

$$\overset{O}{\overset{\|}{-C}}-Ar^{a}-OH \qquad (XII)$$

-O-$Ar^b$-OH (XIII)

-NH-$R^5$-OH (XIV)

wobei die Symbole die schon genannte Bedeutung haben, steht, und wobei das mittlere Molekulargewicht $\overline{M}n$ der Verbindungen der Formel (II) nicht größer als 4.000, bevorzugt nicht größer als 2.500 ist,

sowie gegebenenfalls

3) 0,001-150 Gew.-%, bezogen auf die Summe der Gewichte der Komponenten 1) und 2), an üblichen Zusatzstoffen, wobei die Zusatzstoffe wahlweise auch ganz oder teilweise schon in der unter 1) genannten Polyamiden enthalten sein können,

hergestellt werden.

2. Leichtfließende Polyamid-Formmassen nach Anspruch 1, dadurch gekennzeichnet, daß als Bausteine für die flüssig kristallinen Verbindungen der Formel (II) bevorzugt 4-Hydroxy-3-methylbenzoesäure, 4-Hydroxy-3-methoxybenzoesäure, 4-Hydroxybenzoesäure, 3-Hydroxybenzoesäure, 6-Hydroxy-2-naphthoesäure, Hydrochinon, 4,4'-Dihydroxydiphenyl, 4,4'-Dihydroxydiphenylether, 4,4'-Dihydroxydiphenylethan, 4,4'-Dihydroxydiphenoxyethan, 3,5'-Dihydroxydiphenyl, 3,5'-Dihydroxydiphenylether, 1,5-Dihydroxynaphthalin, 2,6-Dihydroxynaphthalin, 1,4-Dihydroxynaphthalin, Methylhydrochinon, Phenylhydrochinon, 2,2'-Dimethyl-4,4'-dihydroxydiphenyl, 3,3',5,5'-Tetramethyl-4,4'-dihydroxydiphenyl, 1,2-(2-Chlor-4-hydroxyphenoxy)-ethan, 4-Methoxy-2,6-dihydroxynaphthalin, Resorcin, 3,4'-Dihydroxydiphenyl, 1,6-Dihydroxynaphthalin, 1,7-Dihydroxynaphthalin, 2,7-Dihydroxynaphthalin, 4-Chlorresorcin, 4-Phenylresorcin, 4-Ethoxyresorcin, 2,5-Dichlor-1,6-dihydroxynaphthalin und 4-Methoxy-2,7-dihydroxynaphthalin, 1,4-Naphthalindicarbonsäure, 1,5-Naphthalindicarbonsäure, 2,6-Naphthalindicarbonsäure, Biphenyl-4,4'-dicarbonsäure, Biphenyl-3,3'-dicarbonsäure, Diphenoxyethan-4,4'-dicarbonsäure, Diphenylether-4,4'-dicarbonsäure, Methylterephthalsäure, Methoxyterephthalsäure, Chlorterephthalsäure, 4-Chlornaphthalin-2,7-dicarbonsäure, 2,6- oder 2,7-Naphthalindicarbonsäure, Biphenyl-3,4'-dicarbonsäure, 4-Methylisophthalsäure, 5-Methylisophthalsäure, Diphenylether-4,4'-dichlor-3,3'-dicarbonsäure, Iso- und Terephthalsäure, ε-Aminocapronsäure bzw. Caprolactam, ω-Aminoundecansäure, ω-Aminododecansäure bzw. Laurinlactam, 4-Aminocyclohexyl-carbonsäure, 4-Aminobenzoesäure, 6-Amino-2-naphthoesäure, 3-Aminophenol, 4-Aminophenol, 3-Amino-2-methylphenol, 3-Amino-4-methylphenol, 5-Amino-1-naphthol, 6-Amino-1-naphthol, 5-Amino-2-naphthol, 7-Amino-2-naphthol, 8-Amino-2-naphthol, 6-Amino-2-naphthol und 4'-Amino-1-hydroxybiphenyl, Oxalsäure, Bernsteinsäure, Adipinsäure, Korksäure, Azelainsäure, Trimethyladipinsäure, Sebacinsäure, Dodecandisäure, Dimerfettsäuren, 1,4-Cyclohexandicarbonsäure, Benzoesäure, 4-Methylbenzoesäure, 4-Methoxybenzoesäure und 4-Biphenylcarbonsäure,

und besonders bevorzugt Hydrochinon, 4,4'-Dihydroxybiphenyl, 4-Aminophenol, 3-Aminophenol, 4'-Amino-1-hydroxybiphenyl, 4-Hydroxybenzoesäure, 3-Hydroxybenzoesäure, 6-Hydroxy-2-naphthoesäure, Isophthalsäure, Terephthalsäure, Oxalsäure, Adipinsäure, Sebazinsäure und Dimerfettsäuren, 4-Aminobenzoesäure und 6-Amino-2-naphthoesäure, ε-Aminocapronsäure bzw. Caprolactam, Benzoesäure, 4-Methylbenzoesäure und 4-Biphenylcarbonsäure verwendet werden, und wobei die Additive der Formel (II) durchschnittlich bevorzugt 3-25 und besonders bevorzugt 4-20 aromatische Teilstrukturen enthalten.

3. Leichtfließende Polyamid-Formmassen nach mindestens einem der Ansprüche 1 bis 3, dadurch gekennzeichnet, daß als Polyamid-Komponenten 1), einzeln oder im Gemisch, bevorzugt Polyamid 6, 66, 46, 610, 11, 12, 1012, 1212, 6I6, 6I, 6T/6, 6T6, 6/66-Copolyamide, Copolyamide aus Caprolactam, Isophthalsäure und Isophorondiamin, Copolyamide aus Isophthalsäure (+ ggf. Terephthalsäure), Azelainsäure und 4,4'-Diaminodicyclohexylmethan (bzw. Isomerengemische) und besonders bevorzugt Polyamid 6 und 66 eingesetzt werden.

4. Polyamid-Formmassen nach mindestens einem der Ansprüche 1 bis 4, dadurch gekennzeichnet, daß als polymere Legierungspartner gemäß 3) u.a. Dienkautschuke, Acrylat-Kautschuke, Polyethylen, Polypropylen, Ethylen/Propylen-Copolymere, Ethylen/1-Buten-Copolymere, Ethylen/Propylen-Dien-Terpolymere, Ethylen- und/oder Propylen-Acrylsäure(ester)-Copolymere, Ethylen/Vinylacetat-Copolymere, Polyoctenylene, Polystyrole, Styrol/(Meth)acryl nitrilcopolymere, (Meth)Acrylnitril/Butadien/Styrol-Polymere (ABS), schlagzähe Polystyrole, Polycarbonate, aromatische Polyester(carbonate), Polyester wie z.B. Polyethylenterephthalat, Polysulfone, Polyphenylenoxide, Polyetherketone, Polyethersulfone, Polyarylensulfide, Polyetheretherketone, Polyetherimide, Polyesterimide, Polyamidimide und Polyimide eingesetzt werden, und gegebenenfalls niedermolekulare und/oder polymere Verträglichkeitsvermittler anwesend sind.

**5.** Leichtfließende Polyamid-Formmassen nach Anspruch 1, dadurch gekennzeichnet, daß als Zusatzstoffe gemäß 3) Verstärkungsmaterialien (Glasfasern, Aramidfasern, Kohlenstoffasern, Glaskugeln, $SiO_2$, Kreide, $TiO_2$, Talkum, Kaolin, Glimmer, $TiO_2$ u.a.), Weichmacher, Antioxidantien, Pigmene, Farbstoffe, Bewitterungsstabilisatoren (bzw. Stabilisatorkombinationen), Gleitmittel, Fließhilfsmittel, Entformungsmittel, Nukleierungsmittel, die Wasseraufnahme verringernde Zusätze (bevorzugt Monophenole, Bisphenole und (Alkyl)phenol-Formaldehyd-Kondensate), Flammschutzmittel, polymere Legierungspartner u.a.m. eingesetzt werden können, wobei zur Herstellung von Polyamid-Legierungen immer mindestens ein polymerer Legierungspartner nach Anspruch 4 als Zusatzstoff gemäß 3) eingesetzt werden muß, und wobei die Zusatzstoffe gemäß 3) unabhängig voneinander für sich oder in Form eines Konzentrats zudosiert werden oder auch nach Vorvermischung mit dem Polyamid als in diesem enthalten zudosiert werden können.

**6.** Verfahren zur Herstellung der Polyamid-Formmassen nach Anspruch 1, dadurch gekennzeichnet, daß mindestens ein Polyamid gemäß 1), mindestens ein flüssig kristallines Additiv (I) und/ oder (II) gemäß 2) sowie gegebenenfalls die weiteren Zusatzstoffe gemäß 3), wobei zur Herstellung von Polyamid-Legierungen immer mindestens ein polymerer Legierungspartner als Zusatzstoff gemäß 3) eingesetzt wird, und wobei die gegebenenfalls miteinzusetzenden Zusatzstoffe 3), auch polymere Legierungspartner, unabhängig voneinander für sich oder als Konzentrat eingesetzt werden können, oder auch schon in den Polyamiden 1) enthalten sein können, in der Schmelze, bevorzugt in Extrudern, vermischt werden, und die Schmelze in bekannter Weise aufgearbeitet wird.

**7.** Formkörper, Folien, Fasern, Verbundwerkstoffe und andere Gegenstände aus Polyamid-Formmassen nach mindestens einem der Ansprüche 1 bis 6.

**8.** Flüssig kristalline Verbindungen der Formeln (I) und (II) nach Anspruch 1.

**9.** Flüssig kristalline Verbindungen der Formeln (I) und (II) nach Ansprüchen 1 und 8, dadurch gekennzeichnet, daß als bevorzugte bzw. besonders bevorzugte Bausteine die nach Anspruch 2 eingesetzt werden.

**10.** Verwendung von niedermolekularen, flüssig kristallinen Estern und/oder Esteramiden 2) der allgemeinen Formel (I) und/oder oligomeren, flüssig kristallinen Estern und/oder Esteramiden der Formel (II) nach Ansprüchen 8 und 9 als flüssig kristalline Fließverbesserer für Polyamide in Mengen von 0,1 bis 15 Gew.-%, bezogen auf die Summe der Gewichte von 1) und 2).